# EUROPEAN PATENT APPLICATION

(11) **EP 2 415 867 A1**
(43) Date of publication of application: **08.02.2012**
(21) Application number: 10758561.4
(22) Date of filing: 26.03.2010
(51) Int. Cl.: C12N 15/09, C12M 1/00, G06F 19/00, C12Q 1/68

(54) **METHOD FOR DESIGNING PRIMER FOR SELEX METHOD, METHOD FOR PRODUCING PRIMER, METHOD FOR PRODUCING APTAMER, DEVICE FOR DESIGNING PRIMER, AND COMPUTER PROGRAM AND RECORDING MEDIUM FOR DESIGNING PRIMER**

(30) Priority: 01.04.2009 JP 2009089607
(71) Applicant: NEC Soft, Ltd., Tokyo 136-8627 (JP)
(72) Inventor: AKITOMI Jou, Tokyo 136-8627 (JP); KATOU Shintarou, Tokyo 136-8627 (JP); YOSHIDA Yoshihito, Tokyo 136-8627 (JP)
(74) Representative: Vossius & Partner
(86) International application number: PCT/JP2010/055345
(87) International publication number: WO 2010/113789

(57) **Abstract**

A method for designing primers, being capable of efficiently designing primers for a SELEX method is provided. The method for designing primers of the present invention includes the steps of: (S1) generating candidate primer sequences; (S2) evaluating the candidate primer sequences according to a predetermined criterion so as to select candidate primer sequences; (S3) based on the selected candidate primer sequences, generating random pools each containing a plurality of nucleic acid sequences that include random sequences and the candidate primer sequences; (S4) predicting a structure of each of the nucleic acid sequences in each of the random pools and evaluating the predicted structure according to a predetermined criterion so as to select a random pool; and (S5) determining that the candidate primer sequences in the selected random pool are employed as primer sequences. The respective steps are carried out using a computer.

## Description

### Technical Field

The present invention relates to a method for designing primers for a SELEX method, a method for producing the primers, a method for producing aptamers, a system for designing the primers, and a computer program and a recording medium for designing the primers.

### Background Art

An aptamer is a nucleic acid ligand that specifically binds to a target substance. A concept of the aptamer was reported by GOLD et al. for the first time in 1990, and the aptamer is obtained by Systematic Evolution of Ligands by Exponential Enrichment (SELEX) method (Patent Document 1 and Non-patent Document 1). The SELEX method is a method in which a series of steps of: immobilizing a target substance on a carrier such as a bead; adding a nucleic acid library such as a DNA library or an RNA library thereto; collecting nucleic acids binding to the target substance; amplifying the collected nucleic acids; and adding again the amplified nucleic acids to the target substance is repeated a total of about 10 times, so that nucleic acids each having high specificity and a high bonding strength to the target substance is concentrated. then base sequences of the nucleic acids are determined, and thus aptamers are obtained. It is expected to find applications of aptamers to pharmaceuticals and sensors. For example, a pegaptanib sodium injection that is a therapeutic agent of angiogenetic age-related macular degeneration (AMD) was developed as a pharmaceutical containing an aptamer and has been put to practical use.

In the SELEX method, it is necessary to design primers in order to amplify nucleic acids. Also in a gene amplification method typified by a PCR, it is necessary to design primer, and many software programs to assist designing primers have been developed. However, there is no software program to assist designing primers for a SELEX method. Moreover, unlike designing primers for the gene amplification method, conducted with predicting gene sequences, designing primers for a SELEX method has no information by which gene sequences can be predicted, so that it is necessary to manually take processes of trial and error with calculating secondary structures, Tm values, and the like from nothing. In addition, designed primers do not always serve as desired. As described above, designing primers for a SELEX method has a problem in that labor and time are required, a cost thereof and uncertainty are involved, and efficiency is poor.

### Prior Art Documents

### Patent Document

Patent Document 1: Japanese Patent No. 2763958

### Non-Patent Document

Non-patent Document 1: Science, (1990), Vol. 249, pp. 505-510

### Summary of Invention

### Problem to be Solved by the Invention

Hence, the present invention is intended to provide a method for designing primers, being capable of efficiently designing primers for a SELEX method, a method for producing the primers, a method for producing aptamers, a system for designing the primers, and a computer program and a recording medium for designing the primers.

### Means for Solving Problem

The method for designing primers for a SELEX method of the present invention includes steps of: generating candidate primer sequences; evaluating the candidate primer sequences according to a predetermined criterion so as to select candidate primer sequences; based on the selected candidate primer sequences, generating random pools each containing a plurality of nucleic acid sequences that include random sequences and the candidate primer sequences; predicting a structure of each of the nucleic acid sequences in each of the random pools and evaluating the predicted structure according to a predetermined criterion so as to select a random pool; and determining that the candidate primer sequences in the selected random pool are employed as primer sequences. The respective steps are carried out using a computer.

The method for producing primers for a SELEX method of the present invention includes: the steps of designing primers; and based on sequences of the primers designed in the step of designing primers, synthesizing primers. The step of designing primers is carried out by the method for designing primers of the present invention.

The method for producing aptamers of the present invention includes producing aptamers by a SELEX method using primers produced by the method for producing primers of the present invention.

The system for designing primers for a SELEX method of the present invention includes: a data processing unit; a storage unit; an input unit; and an output unit. The data processing unit includes: a candidate primer sequence generation section of generating candidate primer sequences; a candidate primer sequence selection section of evaluating the candidate primer sequences according to a predetermined criterion so as to select candidate primer sequences; a random pool generation section of, based on the selected candidate primer sequences, generating random pools each containing a plurality of nucleic acid sequences that include random sequences and the candidate primer sequences; a random pool selection section of predicting a structure of each of the nucleic acid sequences in each of the random pools and evaluating the predicted structure according to a predetermined criterion so as to select a random pool; and a primer sequence determination section of determining that the candidate primer sequences in the selected random pool are employed as primer sequences.

The computer program for designing primers for a SELEX method of the present invention is capable of operating the method for designing primers of the present invention.

The recording medium of the present invention stores the computer program of the present invention.

### Effects of the Invention

According to the present invention, it is possible to design primers that suit on the purpose through computer simulations prior to an actual synthesis of primers. Therefore, it becomes possible to reduce labor, time, and a cost and to efficiently design primers with superior certainty. Moreover, according to the present invention, it is possible to control a secondary structure of each nucleic acid in a random pool. Therefore, for examples, it is possible to control structure of aptamers, and it becomes possible to design a random pool with reference to the structures of known aptamers. Thus, an improvement in efficiency of obtaining intended aptamers can be expected.

### Brief Description of Drawings

[FIG. 1] FIG. 1 is a configuration diagram showing a configuration of an example of a system for designing primers of the present invention.
[FIG. 2] FIG. 2 is a flowchart showing an example of a method for designing primers of the present invention.
[FIG. 3] FIG. 3 is a configuration diagram showing a configuration of another example of the system for designing primers of the present invention.
[FIG. 4] FIG. 4 is an explanatory diagram showing a state where the system for designing primers in the another example connects to a server through a communication network.
[FIG. 5] FIG. 5 is a classification diagram of secondary structures of nucleic acids by graph structures.
[FIG. 6] FIG. 6 is a graph showing distributions of the numbers of vertexes of secondary structures of only random regions (sequences) in the respective random pools of the example of the present invention.
[FIG. 7] FIG. 7 is a graph showing distributions of the numbers of vertexes of secondary structures of all nucleic acid sequences in the respective random pools of the example of the present invention.
[FIG. 8] FIG. 8 is a graph showing distributions of classes of secondary structures of only random regions (sequences) in the respective random pools of the example of the present invention.
[FIG. 9] FIG. 9 is a graph showing distributions of classes of secondary structures of all nucleic acid sequences in the respective random pools of the example of the present invention. Description of Embodiments

Next, the present invention is described with reference to examples.

### (Embodiment 1)

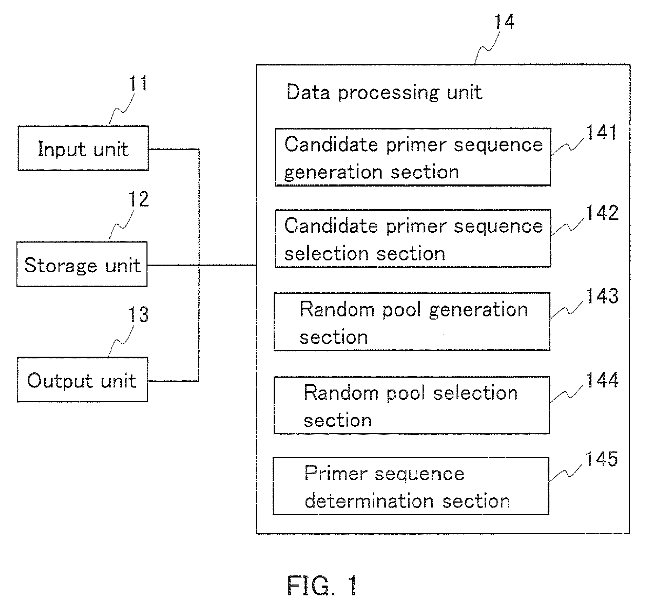

A configuration of an example of the system for designing primers (hereinafter also referred to as "primer design system") of the present invention is shown in FIG. 1. As shown in FIG. 1. the system of the present embodiment includes, as main components, an input unit 11, a storage unit 12, an output unit 13, and a data-processing unit 14, The input unit 11 is for inputting information into the data-processing unit 14, and examples thereof include keyboards, key sheets, mice, terminals such as USB and the like, drives such as a CD and a CD-R. The storage unit 12 is for storing; various types of information such as information on conditions for designing primers, random pools, and designed primer sequences: and various programs and is, for example, configured by combining with a flash memory, a RAM, a ROM, or the like as appropriate. The output unit 13 is for outputting information on designed primer sequences and other information, and examples thereof include displays and printers. The data-processing unit 14 is for conducting a series of processes of designing primers, and can be, for example, a central processing unit (CPU). The data-processing unit 14 of the present embodiment includes a candidate primer sequence generation section 141, a candidate primer sequence selection section 142, a random pool generation section 143, a random pool selection section 144, and a primer sequence determination section 145. In the present invention, the primer design system may be configured in one set of computer or configured in multiple sets of computer.

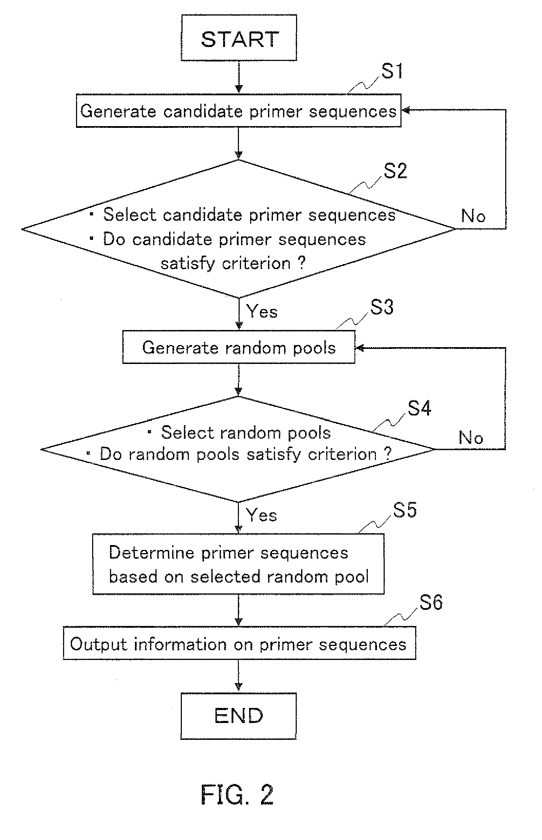

In the present embodiment, the primer design system can design primers according to a flowchart shown in FIG. 2 as follows.

### (Generation of candidate primer sequences: S 1)

Candidate primer sequences are generated by a candidate primer sequence generation section. Primer sequences are combinations of bases of A, G, C, T, and U, for example. In the case where a primer sequence is DNA, T is used, and in the case where a primer sequence is RNA, U is used. It is necessary to generate at least two types of sequences on 3' end side and 5' end side as the candidate primer sequences. The length (number of bases) of each of the candidate primer sequences is not particularly limited, and is in the range from 10 to 100 bases, preferably from 15 to 50 bases, and more preferably from 20 to 30 bases. The candidate primer sequences may be generated randomly or generated with reference to a database of known primers. Moreover, the candidate primer sequences may be input by the input unit 11.

### (Selection of candidate primer sequences: S2)

The generated candidate primer sequences are evaluated according to a predetermined criterion so as to select candidate primer sequences satisfying the predetermined criterion. In this case, when the candidate primer sequences do not satisfy the predetermined criterion (No), candidate primer sequences are again generated, and when they satisfy the predetermined criterion (Yes), the next step is conducted. Examples of the predetermined criterion include a secondary structure forming ability of a primer, a Tm value, a sodium concentration, a GC content, an variation range of the GC content, a nucleic acid concentration, a balance between G and C in a total GC content, and a balance between A and T in a total AT content. The secondary structure forming ability of a primer can be, for example, represented by Max free energy (a threshold value of free energy that is admissible in a primer structure). In order to evaluate the candidate primer sequences, a software program of designing primers for a gene amplification can be used. As such a software program, a commercially available product or a software program posted on the Internet may be used. Many of these software programs of designing primers for a gene amplification can generate primer sequences. When such software programs are used, it is possible to simultaneously conduct generation (S1) and a selection (S2) of candidate primer sequence.

### (Generation of random pools: S3)

The random pools are mixtures (libraries) each containing a plurality of nucleic acid sequences that include random sequences and the candidate primer sequences. The random sequences are candidate aptamer sequences and are each a sequence in which bases of A, G, C, and U or A, G, C, and T are randomly linked. The length (the number of bases) of each of the random sequences is, for example, in the range from 20 to 120 bases, preferably from 30 to 80 bases, and more preferably from 30 to 40 bases. The number of types of the nucleic acid sequences contained in each of the random pools is, for example, in the range from 4²⁰ to 4¹²⁰ (about from 10¹² to 10⁷²) and preferably from 4³⁰ to 4⁶⁰ (about from 10¹⁸ to 10³⁶).

The nucleic acid sequences contained in each of the random pools may further include fixed sequences opposite to the random sequences. The fixed sequences can be, for example, sequences in each of which bases of G or C are linked. For example, a sequence of GGGG may be added to the terminal side of each of candidate forward (Fw) primer sequences, and a sequence of CCCC may be added to the head of each of candidate reverse (Rv) primer sequences.

### (Selection of random pools: S4)

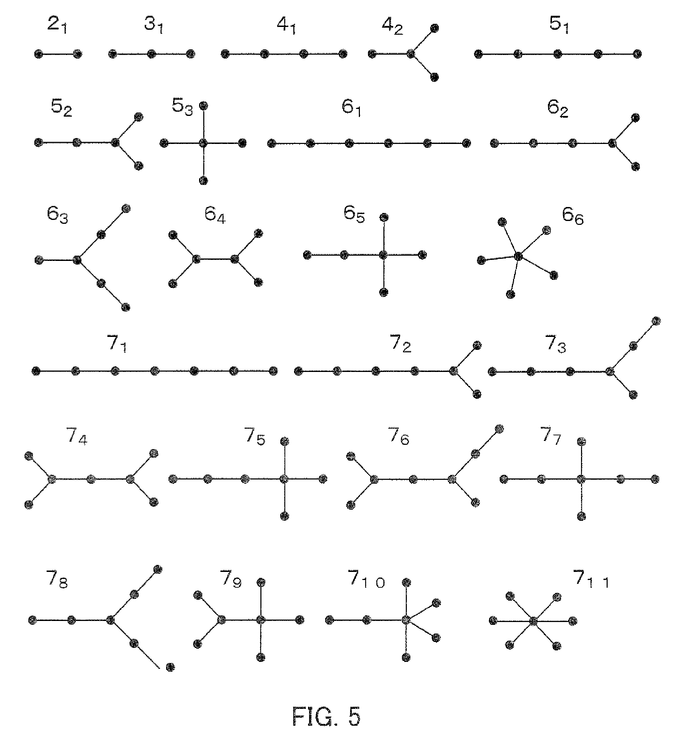

A structure of each of the nucleic acid sequences in each of the random pools is predicted, and the predicted structure is evaluated according to a predetermined criterion so as to select a random pool containing nucleic acid sequences each having a predicted structure satisfying the predetermined criterion. In order to predict the structure, a known software program of predicting a structure of DNA, RNA, or the like can be used. As the criterion, variety of secondary structures can be used. As the variety of secondary structures, classification by graphic structures can be used (GEVERTZ J. et.al. RNA 2005; 11; 853-863). Classification of secondary structures by graphic structures is shown in FIG. 5. In the classification shown in FIG. 5, a stem is represented by a line (-), and a loop is represented by a dot (·). FIG. 5 shows graph structures in each of which the number of dots is 7 or less. When the structure does not satisfy the predetermined criterion (No), the step of generating random pools is conducted again, and when the structure satisfies the predetermined criterion (Yes), the next step is conducted.

### (Determination of primer sequences: S5)

It is determined that candidate primer sequences used in the selected random pool are employed as primer sequences. Information on the determined primer sequences is output from an output unit such as a display (S6). Instead of this, the information on the determined primer sequences may be stored in a storage unit.

### (Embodiment 2)

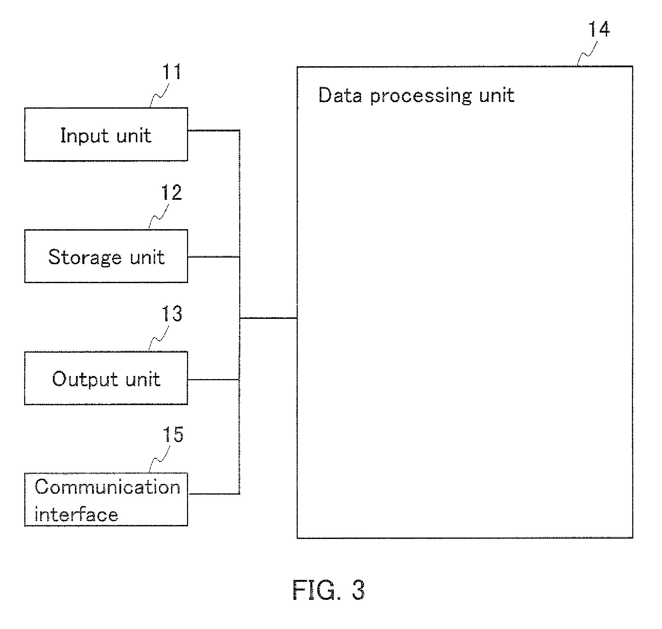

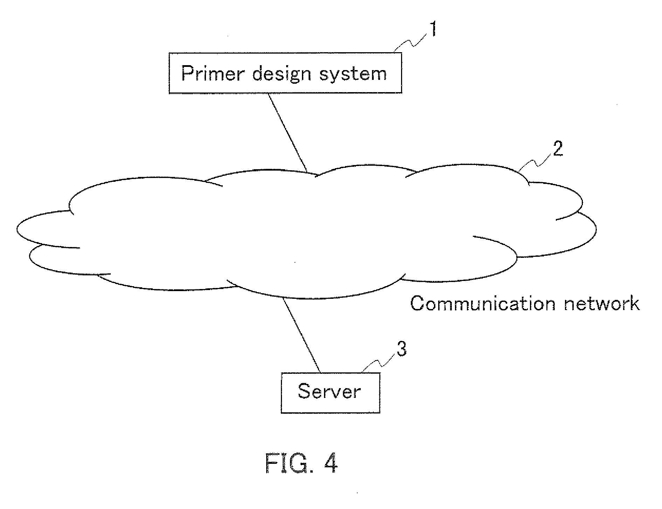

An example of a primer design system including a communication interface (communication unit) is described. A configuration of the system of the present embodiment is shown in FIG. 3. In FIG. 3, identical parts to those shown in FIG. 1 are indicated with identical numerals. As shown in FIG. 3, main components of the system of the present embodiment are the same as those of the system of Embodiment 1 except that the system of the present embodiment includes a communication interface, so that it is possible to connect to a communication network such as the Internet. As shown in FIG. 4, a system 1 of the present embodiment is connectable to a server 3 that is arranged outside of the system 1 through a communication network 2 such as the Internet. Therefore, for example, it is possible to carry out various steps of generating candidate primer sequences, evaluating candidate primer sequences, generating random pools, and predicting a structure of each of nucleic acid sequences in each of random pools by a program in a site of the server that is outside of the system. Thus, according to the system of the present embodiment, it is possible to operate at least one section selected from the group consisting of the candidate primer sequence generation section, the candidate primer sequence selection section, the random pool generation section, the random pool selection section, and the primer sequence determination section by a program in the site of the server that is outside of the system. In this case, the data processing unit in the system of the present embodiment may not include any of the sections operated by the program in the server that is outside of the system.

### (Embodiment 3)

The present embodiment shows a method of producing primers based on primer sequences designed by the method for designing primers of the present invention. The method for designing primers is the same as described above. As a method for synthesizing primers, a known method can be used. The method for synthesizing primers can be, for example, specifically a method in which primers are chemically synthesized from terminal bases using dNTP or the like as a material by a DNA synthesizer or an RNA synthesizer. As the DNA synthesizer or the RNA synthesizer, a commercially available product may be used.

### (Embodiment 4)

The present embodiment shows an example of a method for producing aptamers by a SELEX method using primers produced by the method for producing primers of the present invention. The method for producing primers is the same as described above. The SELEX method is described below.

### (Aptamer)

In the present invention, the aptamers are nucleic acid molecules each of which can specifically bind to a particular target substance, and examples thereof include single-stranded nucleic acids such as single-stranded RNAs and single-stranded DNAs and double-stranded nucleic acids such as double-stranded RNAs and double-stranded DNAs. When nucleic acid aptamers are the latter of double-stranded nucleic acids, it is preferred that each of the double-stranded nucleic acids is caused to be single-stranded nucleic acids by denaturation or the like prior to the use thereof, for example. Each of the nucleic acid aptamers may have a secondary structure formed by self annealing. The secondary structure can be, for example, a stem-loop structure.

In the present invention, the nucleic acid aptamers may have, for example, naturally-derived nucleic acid sequences or synthesized nucleic acid sequences. A method for synthesizing nucleic acid aptamers is not at all limited and can be, for example, a method in which nucleic acid aptamer are chemically synthesized from terminal bases using dNTP or the like as a material by a DNA synthesizer or an RNA synthesizer. Examples of the nucleic acid aptamers include DNAs and RNAs, and each of the nucleic acid aptamers may contain a peptide nucleic acid such as PNA, for example. Each of the a nucleic acid aptamer may contain a natural nucleic acid (non-artificial nucleic acid) including A, C, G, T, U, or the like or a artificial nucleic acid including 2'-fluorouracil, 2'-aminouracil, 2'-O-methyluracil, 2-thiouracil, or the like.

A method for producing nucleic acid aptamers by a SELEX method is not particularly limited and can be conducted as follows, for example. First, a nucleic acid pool containing a plurality of nucleic acids is prepared, and a nucleic acid library is caused to bind to (associated with) a target substance. Thus, a complex between the nucleic acid pool and the target substance is formed. Then, only nucleic acid probes involved in formation of the complex are collected from the complex. Thus, nucleic acid aptamers that can specifically bind to the target substance can be prepared. A method for preparing nucleic acid aptamers that can specifically bind to the target substance using a SELEX method is described below in detail. The present invention, however, is not at all limited by this.

The nucleic acid pool is, for example, a library (mixture) of nucleic acids each having a. random sequence. Examples of the nucleic acids in the library include polynucleotides such as RNA and DNAs. The random region (sequence) is a region (sequence) in which bases of A, G, C, and U or bases of A, G. C, and T are randomly linked, and the length thereof is, for example, in the range from 20 to 120 mer. The nucleic acid pool includes preferably from 4²⁰ to 4¹²⁰ types (about from 10¹² to 10⁷² types) of nucleic acids, more preferably from 4³⁰ to 4⁶⁰ types (about from 10¹⁸ to 10³⁶ types) of nucleic acids.

Each of the polynucleotides in the nucleic acid pool has primer sequences utilized in a nucleic acid amplification described below, a polymerase recognition sequence recognized by a polymerase, and the like at at least one of the 5'-end and 3'-end of the random sequence. The primer sequences are sequences designed by the above-mentioned method for designing primers. The polymerase recognition region (polymerase recognition sequence) can be decided as appropriate according to the type of polymerase used in a nucleic acid amplification described below of an aptamer preparation. In the case where the nucleic acid pool is an RNA pool, the polymerase recognition sequence is, for example, preferably a DNA-dependent RNA polymerase recognition sequence (hereinafter, also referred to as an "RNA polymerase recognition sequence".), and specifically, a T7 promoter that is a T7 RNA polymerase recognition sequence. A specific example of the RNA pool can be, for example, an RNA pool containing RNAs each having a structure in which, from the 5' end side thereof, the RNA polymerase recognition sequence and the primer sequence (hereinafter, also referred to as a "5'-end side primer sequence".) are linked in this order, the random sequence is linked to the 3' end side of the 5'-end side primer sequence, and the primer sequence (hereinafter, also referred to as a "3'-end side primer sequence".) is linked to the 3' end side of the random sequence. It is preferred that the 5'-end side primer sequence in the RNA is, for example, a sequence complementary to the 3' end of a DNA antisense strand synthesized using the RNA as a template, i.e., a sequence that is the same as a sequence of a primer that can bind to the 3' end of the antisense strand. Moreover, the RNA pool may include nucleic acids each having a region (sequence) that supports the RNA pool to bind to a target substance. Each of the polynucleotides in the nucleic acid pool may have a different random sequence or a random sequence part of which is a common sequence. The respective sequences in each of the polynucleotides may be directly adjoined (linked) to one another or may be indirectly adjoined (linked) through intervening sequences.

A method for preparing the nucleic acid pool is not particularly limited, and a known method can be employed. In the case where the nucleic acid pool is an RNA pool, the nucleic acid pool can be prepared using an initial pool containing DNAs and, as templates, the DNAs, for example. Hereinafter, a DNA strand being a template of RNAs in a nucleic acid pool is also referred to as an antisense strand, and a DNA strand having a sequence of any of the RNAs with U replaced by T is also referred to as a sense strand. It is preferred that the initial pool containing DNAs contains any of DNAs (antisense strands) each obtained by replacing U in a strand complementary to each random region (sequence) in the RNA pool by T and DNA (sense strands) each having a sequence obtained by replacing U in each random region (sequence) by T. A nucleic acid amplification is conducted using each of the DNAs in this initial pool as a template and a DNA-dependent DNA polymerase. Thereafter, a transcription reaction is conducted using each of obtained DNA amplification products as a template and a DNA-dependent RNA polymerase. Thus, a nucleic acid pool containing RNAs is prepared.

It is also possible that a nucleic acid pool containing RNAs is prepared by a nucleic acid amplification through a preparation of an initial pool containing DNAs each obtained by replacing U in each random region of each of the RNAs by T and annealing of primers each having an RNA polymerase recognition sequence and a sequence complementary to a 5'-end side primer sequence, using the initial pool as a template.

Then, the nucleic acid pool and a target sequence react with each other. Thus, a complex between the nucleic acid pool and the target substance is formed. A binding form between the nucleic acid pool and the target substance is not particularly limited and can be, for example, a bond via intermolecular force such as a hydrogen bond. A treatment for binding between the nucleic acid pool and the target substance can be, for example, a method in which the nucleic acid pool and the target substance are incubated for a certain period of time in a solvent. The solvent is not particularly limited and preferably the one can maintain the bond between the nucleic acid pool and the target substance and the like. Examples of the solvent include various buffer solutions.

Subsequently, the complex between the nucleic acid pool and the target substance is collected. A reaction solution in which the nucleic acid pool and the target substance react with each other in order to form a complex contains, besides the complex, a nucleic acid pool (hereinafter referred to as a "unreacted nucleic acid pool") that does not involved in formation of the complex. Therefore, it is preferred that the complex and the unreacted nucleic acid pool in the reaction solution are separated from each other. A method for separating the complex and the unreacted nucleic acid pool from each other is not particularly limited and can be, for example, a method utilizing the difference in adsorbability between the target substance and the nucleic acid pool or the difference in molecular weight between the complex and the nucleic acid pool.

As the former method utilizing the difference in adsorbability, the following method is illustrative. First, a carrier having adsorbability to the target substance and the reaction solution containing the complex are brought into contact with each other. In this case, the unreacted nucleic acid pool is not adsorbed to the carrier. In contrast, the complex between the target substance and the nucleic acid pool is adsorbed to the same. Thus, the unreacted nucleic acid pool and the complex can be separated from each other. Therefore the complex adsorbed to the carrier can be collected after removing the unreacted nucleic acid pool. It is preferred that the carrier is washed in order to completely remove the unreacted nucleic acid pool prior to collection of the complex from the carrier. The carrier having adsorbability to the target substance is not particularly limited and can be selected as appropriate according to the type of the target substance, for example. In the case where the target substance is, for example, a protein such as an antibody, the carrier having the adsorbability can be, for example, a nitrocellulose film.

As the latter method utilizing the difference in molecular weight, a method using a carrier can be illustrative. The carrier can be, for example, a carrier having pores each with a pore size with which the nucleic acid pool is allowed to pass therethrough, but the complex is not allowed to pass therethrough. Utilizing such a carrier, the complex and the unreacted nucleic acid pool can be separated from each other. The separation may be, for example, electrical separation using an agarose gel, a polyacrylamide gel, or the like.

Besides these methods, the method for separating the complex and the unreacted nucleic acid from each other can be, for example, a method using a target substance immobilized on a carrier in formation of a complex. The target substance is previously immobilized on a carrier, and the carrier and the nucleic acid pool are brought into contact with each other. Thus, a complex between the immobilized target substance and the nucleic acid pool is formed. Then, an unreacted nucleic acid pool binding to no immobilized target substance is removed, and thereafter, the complex between the target substance and the nucleic acid pool is dissociated from the carrier. A method for immobilizing the target substance on the carrier is not at all limited, and a known method can be employed. Specifically, the method can be, for example, a method in which the target substance is previously bound to a tag, and a carrier having a ligand with the tag and the target substance binding to the tag are brought into contact with each other. The tag can be, for example, a His-tag. Examples of the ligand include metal ions such as a nickel ion (Ni²⁺) and a cobalt ion (Co2⁺). Specific examples of the carrier include Ni-agarose and Ni-sepharose based on the metal ions.

Then, a nucleic acid pool involved in formation of the complex it collected from the collected complex. The nucleic acid pool involved in formation of the complex can be collected by releasing a bond between the target substance and the nucleic acid pool, for example.

Subsequently, a nucleic acid amplification, of the collected nucleic acid pool involved in formation of the complex, is conducted. A method, for amplifying the nucleic acid pool is not particularly limited, and the nucleic acid pool can be amplified by a known method according to the type of the nucleic acid pool, for example. In the case where the nucleic acid pool is an RNA pool, cDNAs are prepared by a reverse transcription reaction using an RNA-dependent DNA polymerase, then a nucleic acid amplification of DNAs is conducted by a PCR or the live using the each of the cDNAs as a template, thereafter, using each of amplification products thus obtained as a template and using a DNA-dependent RNA polymerase, a transcription of RNAs is conducted. Thus, the RNA pool involved in formation of the complex can be amplified.

When each of the RNAs in the RNA pool contains an RNA polymerase recognition sequence, a 5'-and side primer sequence, a random sequence, and a 3'-end side primer sequence, the nucleic acid amplification can be conducted by an amplification method utilizing these sequences, for example. It is preferred that, in a reverse transcription reaction for preparing the cDNAs using each of the RNAs as a template, a polynucleotide having a sequence complementary to the 3'-end side primer sequence contained in the RNA pool is used as a primer. Further, it is preferred that, in an amplification of DNAs using each of the cDNAs as a template, a polynucleotide having the 5'-end side primer sequence and a polynucleotide having a strand complementary to the 3'-end side primer sequence are used as primers. It is preferred that the former polynucleotide further has the RNApoiymerase recognition sequence on the 5' end side thereof and the 5'-end side primer sequence on the 3' end side thereof. In an amplification of RNAs using each of obtained amplification products of DNAs as a template, a nucleic acid amplification such as a PCR is conducted using each of the DNA amplification products as a template, a 5'-end side primer sequence and the 3'-end side primer sequence in each of the DNAs, and a DNA-dependent DNA polymerase. In this case, it is preferred that, in the amplification, a polynucleotide containing the 5'-end side primer sequence and a polynucleotide containing a strand complementary to the 3'-end side primer sequence are used as primers. Further, it is preferred that the former polynucleotide has the RNA polymerase recognition sequence on the 5' end side thereof and the 5' end side primer sequence on the 3' end side thereof. Then, a transcription reaction in vitro is conducted using each of obtained amplification products as a template, the RNA polymerase recognition sequence in each of the amplification products, and the DNA-dependent RNA polymerase. Thus, a nucleic acid amplification of the RNA pool involved in formation of the complex can be conducted. In each of the amplification products, a DNA. of an antisense strand has an RNA polymerase recognition sequence on the 3' end side thereof, for example. Therefore, the DNA-dependent RNA polymerase is bound to this region, and each of the RNAs can be synthesized using the antisense strand as a template. The RNA-dependent DNA polymerase used in the reverse transcription reaction is not particularly limited, and a reverse transcriptase derived from avian myeloblastosis virus (AMV Reverse Transcriptase) can be used, for example.

The method for amplifying nucleic acids is not particularly limited, and for example, any of a polymerase chain reaction (PCR) and various isothermal amplification methods can be employed. The conditions thereof are also not particularly limited.

As described above, a nucleic acid pool forming a complex with a target substance is collected. Further, as mentioned above, formation of a complex using a target substance, collection of the complex, separation of a nucleic acid pool involved in formation of the complex, an amplification of the separated nucleic acid pool, and the like are repeated. Thus, nucleic acid aptamers having binding properties to the target substance can be eventually obtained.

In the present invention, the target substance is not particularly limited, and examples thereof include an enzyme, an antibody, a protein such as a structural protein, a nucleic acid, lipid, and an organic polymer.

### Example

Next, the example of the present invention is described. Note here that the present invention is not at all limited by the following example.

### (Generation and selection of candidate primer sequences)

Generation and a selection of candidate primer sequences were conducted using, for example, an Oligo Calculator (http://www.genosys.jp/whatsnew/active/active_manual.html, produced by Signa Genosys). A secondary structure forming ability was employed as a main criterion. With respect to the difference between a set of primers, easily forming a secondary structure, and a set of primers, being difficult to form the same, the set of primers, easily forming a secondary structure, was set to ∼ 10 kcal/mol, and the set of primers, being difficult to form a secondary structure, was set to-1 kcal/mol, using a parameter of Max free energy (a threshold value of free energy that is admissible in a primer structure). The other common parameters were set as follows according to a default value of the software program.

(Common parameters)
FwTm limit = 75.0 (Tm value of Fw primer including t7 sequence of about 75)
RvTm limit = 65.0 (Tm value of Rv primer of about 65)
Ct concentration = 1.0 (nucleic acid concentration in PCR of 1.0 µmol/L (µM))
Na⁺ concentration = 50.0 (Na⁺ concentration in PCR of 50.0 mmol/L (mM))
CC% = 0.6 (each GC% of Fw primer except for t7 sequence and Rv primer of about 60%)
GC% range = 0.1 (variation range of the each GC% is admissible to ±10°%, 50% to 70%) Minimum Na%) = 0.4 (balance between G and C in total GC and balance between A and T in total AT contained in Fw primer except for t7 sequence and Rv primer are admissible to G : C = 4 : 6 to 6 : 4 and A : T = 4 : 6 to 6 : 4, respectively)

### (Generation of random pool)

10000 sets of primers, easily forming secondary structures, and 10000 sets of primers, being difficult to form secondary structures, were prepared from the candidate primer sequences generated as mentioned above. Then, 100 random sequences each with the number of bases of 40 were added to each of the sets of primers. Thus, random pools were generated. A sequence of GGGG was added to the terminal on a Fw primer side of each of the generated sets of primers and a sequence of CCCC was added to the head on a Rv primer side of the same, and thus, other sets of primers were prepared. The random sequences were added to each of the set of primers in the same manner as mentioned above. Thus, random pools were generated.

### (Prediction of secondary structures)

In order to predict secondary structures, RNAfold in Vienna Package (for example, http://www.tbi.univie.ac.at/RNA/, in Hofacker, I. L., Nucl. Acids Res. 2003 31: 3429-3431) was used. In order to operate the RNAfold, a -noLP option was added, so that a stem formed by only a pair of bases was not admissible. In order to predict secondary structures of only the random sequences, stems formed by candidate primer sequences in obtained secondary structures were replaced by loops, after obtaining secondary structures by operating the RNAfold and before evaluating variety described below.

### (Evaluation of variety of secondary structures)

An evaluation of variety of secondary structures was conducted according to the article "GEVERTZ J. et.al. RNA 2005; 11: 853-863". The article proposes that result obtained by predicting secondary structures are input, and structure classes of the respective secondary structures are determined, in order to classify structure classes of the respective secondary structures, classification by graph structures, employed in the article, was employed.

### (Evaluation results)

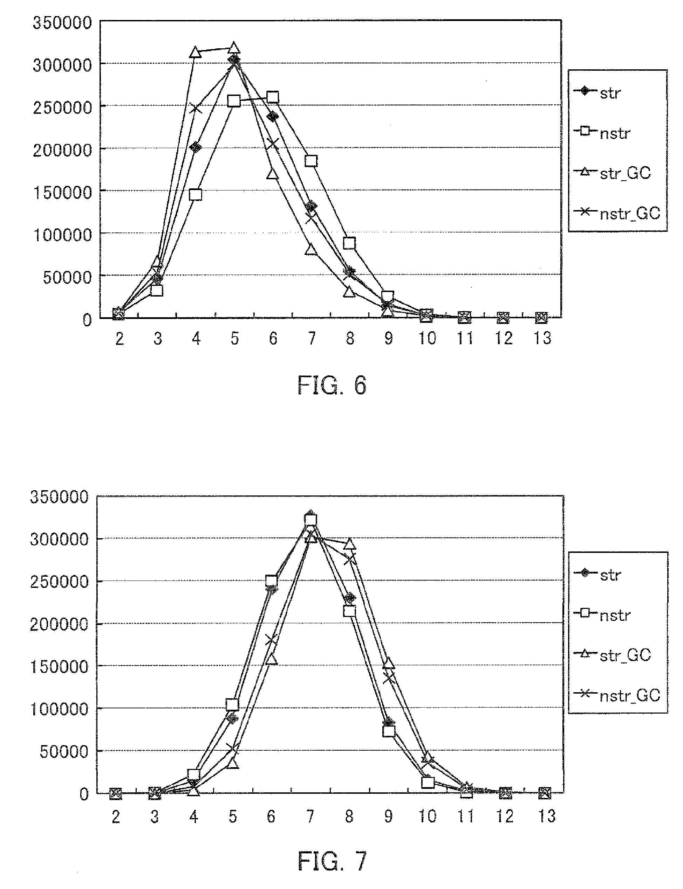

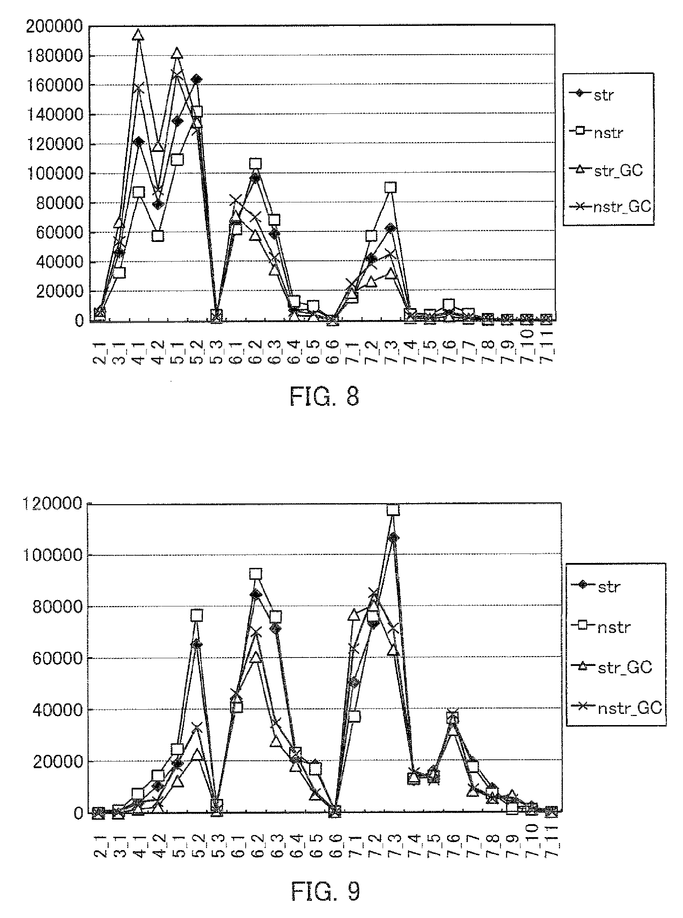

The distributions of the numbers of vertexes of secondary structures in the respective random pools are shown in graphs of FIGs. 6 and 7. In FIGs. 6 and 7, str indicates a random pool containing sets of primers, easily forming secondary structures, nstr indicates a random pool containing sets of primers, being difficult to form secondary structures, str_GC indicates a random pool in which sequences of GGGG + CCCC have been added to each of the sets of primers, easily forming secondary structures, and nstr_GC indicates a random pool in which sequences of GGGG + CCCC have been added to each of the sets of primers, being difficult to form secondary structures. The same applies to FIGs. 8 and 9.

FIG 6 shows distributions of the numbers of vertexes of secondary structures of only random sequences. In FIG. 6. the horizontal axis indicates the number of vertexes, and the vertical axis indicates the number of random sequences. As shown in FIG. 6, distributions of str_GC, nstr_GC, str, and nstr became flatter in this order. This means variety of secondary structures was reduced in this order.

FIG. 7 shows distributions of the numbers of vertexes of secondary structures of all nucleic acid sequences. In FIG. 7, the horizontal axis indicates the number of vertexes, and the vertical axis indicates the number of random sequences. As shown in FIG. 7, the respective distributions are almost the same as each other, and the difference in variety of secondary structures among types of the sets of primers was not found.

The distributions of classes of secondary structures of only random sequences are shown in FIG. 8. In FIG. 8, the horizontal axis indicates a class, and the vertical axis indicates the number of random sequences. The respective numerical numbers of classes correspond to those of classes in FIG. 5. As shown in FIG. 8, the distributions are the same as the respective distributions of the numbers of vertexes, and secondary structures in the random pool using the sets of primers, easily forming secondary structures, were concentrated at around the classes 3 to 5. whereas secondary structures in the random pool using the sets of primers, being difficult to form secondary structures were found at around the classes 6 and 7 as well as at the classes 3 to 5.

FIG. 9 shows distributions of classes of secondary structures of all nucleic acid sequences. In FIG. 9, the horizontal axis indicates a class, and the vertical axis indicates the number of random sequences. The respective numerical numbers of classes correspond to those of classes in FIG. 5. As shown in FIG. 9, the difference in secondary structure according to the types of the sets of primers was not found. However, in the case where the sequences of GGGG and CCCC were added, proportions of linear secondary structures were high.

### (Selection in the case where secondary structures of all nucleic acid sequences are used as a criterion)

As mentioned above, in the case where the sequences of GGGG and CCCC were added, proportions of linear secondary structures were high. Therefore, in the case where the purpose is downsizing nucleic acid sequences in an obtained random pool, the primer sequences to each of which sequences of GGGG and CCCC have been added was employed. In the case where the purpose is not downsizing nucleic acid sequences in an obtained random pool, but increasing variety of secondary structures, the primer sequence to each of which sequences of GGGG and CCCC have not been added is employed.

### (Selection in the case where secondary structures of only random sequences are used as a criterion)

As mentioned above, variety of secondary structures are reduced in order ofstr_GC, nstr_GC, str, and nstr. Therefore, in the case where the purpose is increasing variety of secondary structures, primer sequences being difficult to form secondary structures, to each of which sequences of GGGG + CCCC have not been added, are employed. In contrast, in order for a post-process after obtaining an aptamer to be easily conducted, it is preferred that variety of secondary structures is low. Therefore, for the purpose, primes sequences easily forming secondary structures, to each of which sequences of CGGG + CCCC have been added is employed.

### Industrial Applicability

According to the present invention, primers for a SELEX method can be designed efficiently, whereby an improvement in efficiency of obtaining aptamers can be expected. Therefore, the present invention can be applied to a wide, range of fields such as fields of pharmaceuticals and sensors utilizing aptamers and the like, for example.

### Explanation of reference numerals

- 1: primer design system
- 2: communication network
- 3: server
- 11: input unit
- 12: storage unit
- 13: output unit,
- 14: data processing unit
- 15: communication interface
- 141: candidate primer sequence generation section
- 142: candidate primer sequence selection section
- 143: random pool generation section
- 144: random pool selection section
- 145: primer sequence determination section

## Claims

1. A method for designing primers for a SELEX method, the method comprising the steps of:
generating candidate primer sequences;
evaluating the candidate primer sequences according to a predetermined criterion so as to select candidate primer sequences;
based on the selected candidate primer sequences, generating random pools each containing a plurality of nucleic acid sequences that include random sequences and the candidate primer sequences;
predicting a structure of each of the nucleic acid sequences in each of the random pools and evaluating the predicted structure according to a predetermined criterion so as to select a random pool; and
determining that the candidate primer sequences in the selected random pool are employed as primer sequences, wherein
the respective steps are carried out using a computer.

2. The method according to claim 1, wherein
the predetermined criterion in the step of selecting candidate primer sequences is a criterion associated with a secondary structure forming ability of a primer.

3. The method according to claim 1, wherein
the predetermined criterion in the step of selecting random pools is a criterion associated with variety of possible secondary structures of the random sequences in the nucleic acid sequences.

4. The method according to claim 1, wherein
the nucleic acid sequences further include fixed sequences opposite to the random sequences.

5. A method for producing primers for a SELEX method, the method comprising: the steps of:
designing primers; and
based on sequences of the primers designed in the step of designing primers, synthesizing primers, wherein
the step of designing primers is carried out by the method according to claim 1.

6. A method for producing aptamers, comprising:
producing aptamers by a SELEX method using primers produced by the method according to claim 5.

7. A system for designing primers for a SELEX method, comprising:
a data processing unit;
a storage unit;
an input unit; and
an output unit, wherein
the data processing unit comprises:
a candidate primer sequence generation section of generating candidate primer sequences;
a candidate primer sequence selection section of evaluating the candidate primer sequences according to a predetermined criterion so as to select candidate primer sequences;
a random pool generation section of, based on the selected candidate primer sequences, generating random pools each containing a plurality of nucleic acid sequences that include random sequences and the candidate primer sequences;
a random pool selection section of predicting a structure of each of the nucleic acid sequences in each of the random pools and evaluating the predicted structure according to a predetermined criterion so as to select a random pool; and
a primer sequence determination section of determining that the candidate primer sequences in the selected random pool are employed as primer sequences.

8. The system according to claim 7, wherein
the predetermined criterion in the step of selecting candidate primer sequences is a criterion associated with a secondary structure forming ability of a primer.

9. The system according to claim 7, wherein
the predetermined criterion in the step of selecting random pools is a criterion associated with variety of possible secondary structures of the random sequences in the nucleic acid sequences.

10. The system according to claim 7, wherein
the nucleic acid sequences further include fixed sequences opposite to the random sequences.

11. The system according to claim 7, further comprising:
a communication unit, wherein
the system is connectable to a server through a communication network that is outside of the system by the communication unit, and
a program in the server can operate at least one section selected from the group consisting of the candidate primer sequence generation section, the candidate primer sequence selection section, the random pool generation section, the random pool selection section, and the primer sequence determination section.

12. The system according to claim 11, wherein
the data processing unit does not comprise the at least one section operated by the program in the server.

13. A computer program for designing primers for a SELEX method, being capable of operating the method according to claim 1 on a computer.

14. A recording medium storing the computer program according to claim 13.
